Europäisches Patentamt

(19) European Patent Office

Office européen des brevets

(11) Numéro de publication: **0 050 062**
**B1**

(12) **FASCICULE DE BREVET EUROPEEN**

(45) Date de publication du fascicule du brevet: **04.12.85**

(51) Int. Cl.⁴: **A 61 F 5/01,** A 41 B 13/02

(21) Numéro de dépôt: **81401475.9**

(22) Date de dépôt: **23.09.81**

(54) **Lange destiné à prévenir ou guérir une anomalie d'une hanche ou des deux hanches d'un enfant en bas âge.**

(30) Priorité: **15.10.80 FR 8022520**
**29.12.80 FR 8027989**

(43) Date de publication de la demande:
**21.04.82 Bulletin 82/16**

(45) Mention de la délivrance du brevet:
**04.12.85 Bulletin 85/49**

(84) Etats contractants désignés:
**FR**

(56) Documents cités:
**BE-A- 636 931**
**DE-A-2 217 289**
**DE-B-1 240 222**
**FR-A-2 050 637**
**FR-A-2 464 703**
**US-A-2 935 984**

(73) Titulaire: **Lambert, Jacques**
**29 rue de la Gare**
**F-59232 Vieux-Berquin (Nord) (FR)**

(72) Inventeur: **Lambert, Jacques**
**29 rue de la Gare**
**F-59232 Vieux-Berquin (Nord) (FR)**

(74) Mandataire: **Lepage, Jean-Pierre**
**Cabinet Lepage & Aubertin Innovations et**
**Prestations 23/25, rue Nicolas Leblanc**
**F-59011 Lille Cédex 1 (Nord) (FR)**

Courier Press, Leamington Spa, England.

## Description

L'invention est relative à un lange pour enfant en bas âge, et plus particulièrement pour enfant de moins de quatre mois environ, et de toute façon pour un enfant qui n'est pas encore capable de marcher.

Le lange selon la présente invention est destiné à prévenir ou guérir une anomalie d'une hanche ou des deux hanches de l'enfant, telle qu'une dysplasie ou subluxation de la hanche. Un enfant, au moment de sa naissance, peut présenter de telles anomalies, au niveau de l'une de ses hanches, ou de ses deux hanches, ou celles-ci peuvent apparaître quelques temps après sa naissance. Elles sont éventuellement décelables par un examen clinique et radiologique, et généralement sans conséquence lorsqu'elles sont traitées avec succès dès leur diagnostic.

Les moyens de traitement actuellement existants consistent à maintenir les cuisses de l'enfant dans une position dite en abduction, c'est-à-dire écartées l'une de l'autre et fléchies par rapport au basin.

Deux types de dispositifs actuellement existants permettent de maintenir les cuisses de l'enfant dans une telle position. Il existe tout d'abord des dispositifs rigides, qui sont disposées au-dessus des vêtements de l'enfant, tels que par exemple la culotte de Becker, le harnais de Pavlick, et les attelles de Von Rozen. De tels dispositifs rigides présentent l'inconvénient majeur de maintenir les membres de l'enfant dans une position forcée, d'où il peut résulter des cas plus ou moins graves d'ostéochondrite. Par ailleurs, leur mise en place et leur réglage, tenant compte de la morphologie de l'enfant, sont particulièrement délicats, et sont affaire de spécialistes. Ils sont donc d'un emploi peu commode, en particulier pour une maman.

Un autre type de dispositifs pour diminuer les risques de lésions irréversibles qu'est l'ostéochondrite, utilise des matériaux plus souples, tels que des langes en coton, présentant éventuellement une découpe particulière, des couches traditionnelles superposées, etc. On peut citer à titre d'exemple, une méthode pour langer un enfant connuesous le nom méthode de Saint-Vincent de Paul. Avec de tels dispositifs, le maintien en position de cuisses de l'enfant est obtenu en superposant des langes traditionnels, et/ou en disposant entre les cuisses de l'enfant des langes roulés.

La mise en place de tels dispositifs est donc relativement compliquée, étant donné qu'ils mettent en oeuvre plusieurs éléments indépendants, qu'il est nécessaire de positionner les uns par rapport aux autres, et par rapport au corps de l'enfant.

En outre, les dispositifs actuellement existants sont placés au-dessus des vêtements ou du langeage traditionnel de l'enfant.

Il faut par ailleurs remarquer, d'un manière générale, que le maintien des cuisses de l'enfant en position dite d'abduction doit être permanent, au moins jusqu'à la disparition des anomalies de ses hanches. Les dispositifs actuellement existants sont donc d'un emploi peu facile pour la mère de l'enfant, et relativement encombrants.

Ils présentent par ailleurs un inconvénient important pour la mère de l'enfant, sur le plan psychologique. En effet, ils sont d'un aspect imposant, et généralement repoussant, en tous les cas en disproportion avec la gravité apparente de l'anomalie des hanches de l'enfant.

En outre, les dispositifs actuellement existants présentent un caractère curatif, et du fait de leur complexité, ne peuvent pas être utilisés à titre préventif. If faut en effet remarquer que langer un enfant en position dite d'abduction dès sa naissance, même si une anomalie de l'une de ses hanches n'est par diagnostiquée, peut prévenir l'évolution de telles anomalies, et surtout d'une innocuité totale pour un enfant présentant des hanches normales.

Le brevet américain No. 2.935.984 décrit un lange d'abduction pour corriger les dysplasies. Ce lange est constitué d'une enveloppe extérieure qui contient tout d'abord un rembourrage ainsi qu'une série de nervures transversales pour améliorer la rigidité.

Le brevet allemand No. 2.217,289 décrit un lange écarteur pour les jambes d'enfants qui ont une luxation de la hanche. Selon l'invention, il s'agit d'une pièce de toile qui présente des raidisseurs intérieurs que l'on applique au niveau de l'entre-jambe du bébé.

Toutefois, dans ces deux brevets, les langeages décrits sont fort épais, emprisonnent littéralement le nouveau-né, présentent un angle d'abduction imposé par les moyens mis en oeuvre avoisinant les 90°. Par ailleurs, ces langeages orthopédiques sont peu commodes et d'utilisation plus difficile pour la mère de famille.

De plus, il est généralement reconnu que la matériel d'abduction doit être suffisamment souple pour permettre à l'enfant de soulager les zones d'hyperpression et de conserver une bonne vascularisation de la tête fémorale ce qui n'est par le cas dans ce deux brevets antérieurs.

Ainsi, un des buts de la présente invention est de proposer un lange présentant principalement un caractère préventif, vis-à-vis d'une anomalie des hanches d'un nouveau-né, mais également un caractère curatif.

Un autre but de la présente invention est de proposer un dispositif dont la mise en place sur l'enfant est particulièrement simple, même pour une maman sans connaissance médicale particulière.

Un autre but de la présente invention est de proposer un dispositif qui soit peu encombrant, et qui puisse être disposé sous les vêtements de l'enfant, ou intégré à ceux-ci, ce qui, d'un point de vue psychologique, facilite son utilisation.

Un autre but de la présente invention est de proposer un dispositif qui n'entrave pas totalement, en particulier, le mouvement des cuisses de l'enfant, et qui ne la maintienne pas en position forcée.

Un autre but de la présente invention est de proposer un dispositif qui se prête avantageusement à une fabrication en grande série, et qui soit complémentaire ou combiné aux langes actuellement existants, tels que les "pointes", les "couches-culottes", les "change-complets",..

D'autres buts et avantages de la présente invention apparaîtront au cours de la description qui va suivre, qui n'est cependant donnée qu'à titre indicatif, et qui n'a pas pour but de la limiter.

Le lange pour enfant en bas âge destiné à prévenir ou guérir une anomalie d'une hanche ou de deux hanches, telle qu'une dysplasie, une subluxation de la hanche ou une luxation réductible et stable sous langeage en abduction, et constitué par des moyens d'orientation et des moyens de maintien souples se présentant sous la forme d'une pièce de tissue ou de plastique souple comprenant deux extrémités latérales effiliées qui peuvent être nouées autour de la taille de l'enfant, et d'une ceinture sensiblement inextensible les moyens de maintien assurant le maintien en position sur les cuisses et le bassin de l'enfant des moyens d'orientation lesquels servent à positionnen les jambes de l'enfant de façon fléchie à 90 degrés par rapport au bassin et en abduction à un minimum de 60 degrés, ce lange étant caractérisé par le fait que:

— les extrêmités latérales effilée pout prévues pour être mouées dans le des de l'enfant.

— les moyens d'orientation sont du type ischio-sacré et constitués essentiellement d'un élément transversal unique en forme de traverse ou de coussin en materiau rigide ou semirigide,

— ledit élément transversal cintré selon une courbe qui correspond sensiblement à l'angle d'ouverture des cuisses de l'enfant que l'on désire obtenir,

— la longueur de l'élément transversal est prévue sensiblement égale à la distance séparant les genoux de l'enfant lorsque ses cuisses sont en abduction de telle sorte que ledit élément serve d'appui aux faces postérieures des cuisses de l'enfant,

— les moyens de maintien sont conformés de manière, en cours d'utilisation, à appuyer l'élément transversal sur les faces postéro-inférieures des cuisses de l'enfant et orienter ainsi les cuisses sensiblement parallèlement aux extrémités dudit élément.

L'invention sera mieux comprise si l'on se réfère à la description ci-dessous, ainsi qu'aux dessins en annexe qui en font partie intégrante.

La figure 1 représente schématiquement certains éléments constitutifs du lange selon la présente invention, dans une mode non limitatif de mise en oeuvre de celle-ci.

La figure 2 représente un enfant langé avec le dispositif schématisé en figure 1.

La figure 3 schématise certains éléments constitutifs du lange, dans un autre mode non limitatif de mise en oeuvre de l'invention.

La figure 4 représente un enfant langé avec le dispositif de la figure 3.

Le terme "lange" qui est utilisé ici désigne d'un manière générale tout dispositif destiné à envelopper des enfants en bas âge au-dessous de la ceinture. Ce terme doit donc être pris dans un sens large.

Le lange selon la présente invention comprend des moyens d'orientation, et des moyens de maintien. Cependant, comme il apparaîtra ultérieurement, il peut présenter également selon les cas d'autres moyens, tels que des moyens d'absorption des excréments de l'enfant, des moyens d'étanchéité, des moyens destinés à prevenir l'irritation de la peau de l'enfant, etc. . . .

En outre, dans la suite, le terme "longitudinal" qualifie, pour les différents moyens, la direction orientée de la partie avant vers la partie arrière du lange, et inversement. La terme "transversal" qualifie une direction sensiblement perpendiculaire à la direction longitudinale.

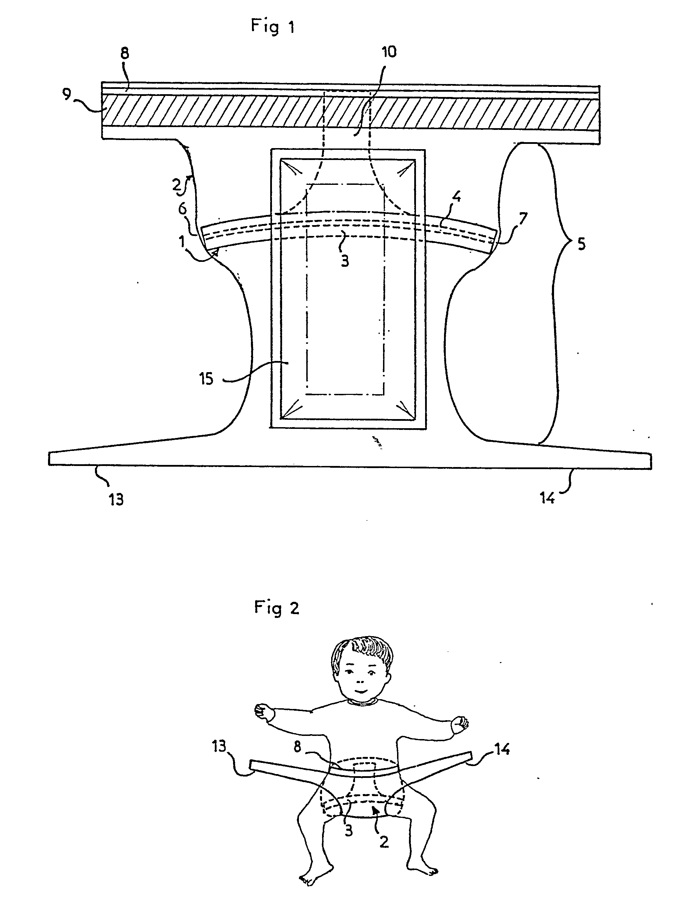

Les figures 1 et 2 illustrent la présente invention dans un mode non limitatif de mise en oeuvre de celle-ci. Ces figures représentent en particulier des moyens d'orientation 1, et des moyens de maintien 2. Les moyens d'orientation 1 sont sensiblement symétriques par rapport à un axe longitudinal.

Les moyens d'orientation 1 sont principalement constitués par une traverse 3, en matériau semi-rigide ou éventuellement rigide, qui s'étend transversalement au lange. De préférence, la traverse 3 est cintrée, selon une courbe qui correspond sensiblement, à l'angle d'ouverture des cuisses de l'enfant que l'on désire obtenir. Egalement, sa section transversale, qui peut être de toute forme appropriée, et par exemple rectangulaire, elliptique, ovale, est très aplatie, sa plus grande dimension se situant sensiblement dans le plan de la figure 1.

Par ailleurs, le longueur de la traverse 3 est déterminée de manière à ce que, lorsque le lange est en position sur l'enfant, les faces postérieures de ses cuisses prennent appui sur elle. Ainsi, cette longueur est égale ou légèrement inférieure à la distance séparant les deux genoux de l'enfant, lorsque ses cuisses sont das dans la position désirée.

La semi-rigidité, ou éventuellement la rigidité, de la traverse 3 est obtenue en réalisant cette traverse dans un matériau semi-rigide ou rigide, ou en renforçant celle-ci par une nervure 4.

La lange selon la présente invention comprend par ailleurs des moyens de maintien 2. Dans la figure 1, ces moyens sont schématiqués par une pointe traditionnelle, en tout matériau approprié, et par exemple en polychlorure de vinyl lavable.

La zone centrale 5 des moyens de maintien 2 peut être constituée d'une feuille, ou d'une enveloppe à l'intérieur de laquelle sont insérés les moyens d'orientation 1. Cette zone centrale présente une forme connue, adaptée à la morphologie de l'enfant. Cependant, de préférence, tel que cela est schématise dans la figure 1, elle présente deux "bosses" 6 et 7, au niveau de chaque extrémité de la traverse 3.

Les moyens de maintien 2 présentent par ailleurs dans leur partie supérieure ou postérieure, et dans leur partie inférieure ou antérieure, des moyens de fixation au corps de l'enfant. Tels qu'ils sont schématisés en figure 1, ces moyens de fixation consistent, pour la partie supérieure ou postérieure, en une ceinture 8, éventuellement renforcée par un ruban souple sensiblement inextensible 9. La ceinture 8 présente à chacune de ses extrémités des moyens de fermeture complémentaires, tels que des moyens adhésifs, des moyens connus sous le nom de bandes velcro, (Velcro étant une marque déposée), etc ..., qui permettent de préférence un réglage en longueur de la ceinture.

Par ailleurs, le ceinture pourrait comporter dans la zone qui sera applique sur le nombril de l'enfant, une compresse, non représentée en figure 1, destinée à assurer la contention de l'orifice du cordon ombilical.

Dans un mode préférentiel de réalisation, la traverse 3 est positionnée et maintenue par rapport à la ceinture 8 par une languette 10 qui les relie l'une à l'autre.

Dans le mode de réalisation représenté en figure 1, les moyens de maintien comprennent dans leur partie inférieure ou antérieure deux extrémités latérales effilées 13 et 14, aptes à permettre une fixation de cette extrémité du lange par nouage dans le dos de l'enfant, ou par des moyens adhésifs ou autres.

D'autres moyens de fixation des moyens de maintien pourraient être les utilisés à la place de la ceinture 8 et des extrémités latérales effilées 13 et 14, et par exemple un dispositif du type à bretelles.

Tel que cela a été indiqué précédemment, le lange peut présenter d'autres moyens, et par exemple des moyens d'absorption constitués par un tampon absorbant 15 disposé au niveau de la partie centrale du lange. Le tampon 15 peut être intégré aux moyens de maintien et relié à ceux-ci, par exemple, par soudure ou collage. Dans ce cas, le tampon absorbant est situé au-dessus de la traverse 3.

Dans la figure 2, on a représenté un enfant lange avec le dispositif de la figure 1. La ceinture 8 des moyens de maintien 2 a été fermée autour de la taille de l'enfant, la zone centrale 5 été tirée énergiquement en la faisant passer entre les jambes de l'enfant. Il ne reste plus qu'à nouer les extrémités latérales effilées 13 et 14 dans le dos de l'enfant, autour de sa taille. Ces différentes opérations amènent la traverse 3, plus précisément ses extrémités, en contact avec les parties postérieures des cuisses de l'enfant. Les moyens de maintien appuient la traverse sur les cuisses de l'enfant, et réciproquement, orientant ainsi les cuisses sensiblement parallèlement aux extrémités de la traverse.

Les suisses de l'enfant sont ainsi orientées et maintenues dans une position dite en abduction. Cette position peut être définie par le fléchissement des cuisses approximativement à 90 degrés par rapport au bassin, et une ouverture de celle-ci d'environ 120 degrés, c'est-à-dire respectivement écartées d'environ 60 degrés par rapport à leur position groupée.

Il faut remarquer que dans le cas du présent dispositif, les cuisses de l'enfant ne sont pas mises dans une position forcée. L'enfant dispose en effet d'une possibilité relative de mouvements, du fait de la nature semi-rigide des moyens d'orientation, et de la nature souple des moyens de maintien. Les moyens d'orientation et les moyens de maintien agissent en combinaison pour ramener les cuisses de l'enfant dans la position correcte d'abduction, au cas où celles-ci s'en écarteraient. Les moyens d'orientation agissent principalement, dans le cas présent, au niveau de la face postéro-inférieure des cuisses.

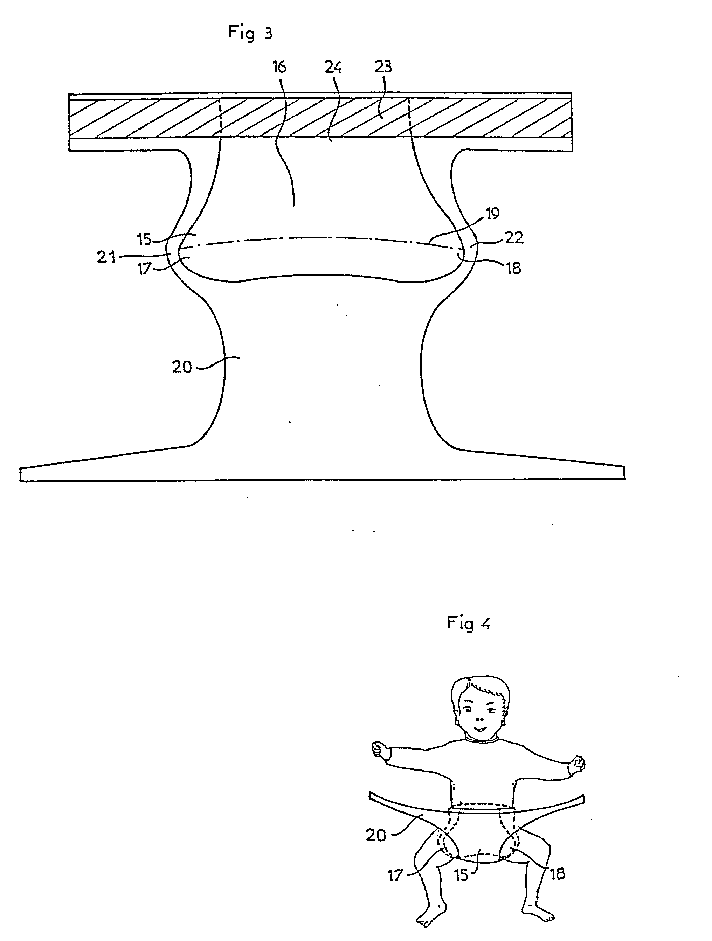

Dans les figures 3 et 4, est représentée une variante du dispositif de la figure 1. Selon cette variante, les moyens d'orientation sont constitués par un coussin 15. Le coussin comprend une zone 16, destinée à être positionée et maintenue sur le bassin de l'enfant, et deux "oreilles" 17 et 18, respectivement destinées à être appliquées et maintenues au niveau de la face postéro-inférieure des cuisses de l'enfant, sensiblement de la même manière que les extrémités de la traverse 3 de la figure 1. De préférence, dans sa partie inférieure, entre les deux oreilles 17 et 18, le coussin pourrait présenter une forme arrondie convexe.

Le coussin 15 est réalisé en un matériau semi-rigide, ou éventuellement rigide. Avantageusement, il pourra présenter une forme incurvée, de manière à constituer sensiblement une cuvette avant ou après mise en place, dans laquelle l'enfant est "assis" en position d'abduction et de flexion. La distance entre les extrémités des deux oreilles 17 et 18 est sensiblement égale, ou inférieure, à la distance entre les deux genoux de l'enfant dans la position en abduction.

Par ailleurs, éventuellement, le coussin pourra être rigidifié par des traverses, telles que par exemple celles schématisées par le trait mixte 19.

Les moyens de maintien 20 sont sensiblement du même type que ceux qui ont été décrits en référence à la figure 1. De préférence, au niveau des deux oreilles 17 et 18, ils présentent des bosses 21 et 22. Le coussin 15 peut être relié à le ceinture sensiblement inextensible 23 des moyens de maintien par une languette 24. Eventuellement, le coussin peut être intégré dans une pouche des moyens de maintien 20.

Comme dans le cas précédent, des moyens d'absorption peuvent être intégrés ou associés aux moyens d'orientation et aux moyens de maintien.

La figure 4 représente un enfant langé avec le dispositif de la figure 3. Le mode de langeage, ainsi que le mode d'action des moyens d'orientation et des moyens de maintient sont sensiblement identiques à ceux du dispositif représenté dans les figures 1 et 2.

Naturellement, à la vue de ce qui précède, d'autres moyens d'orientation semi-rigides agissant sur la face postéro-inférieure des cuisses de l'enfant, c'est-à-dire retenant les cuisses depuis leur face postérieure, pourraient être utilisés sans pour autant sortir du cadre de l'invention. Les moyens d'orientation agissant sur la face postéro-inférieure des cuisses sont du type ischio-sacré.

D'autre part, d'une manière générale, des moyens d'étanchéité et d'autres moyens pourraient être associés ou intégrés aux dispositifs qui viennent d'être décrits. En d'autres termes, les moyens de maintien pourraient être du type actuellement connu sous le nom de "change-complet", "change-slip", "change-culotte", etc. . . .

Les différents modes de réalisation du lange selon l'invention, qui viennent d'être décrits, se prêtent particulièrement bien à une fabrication en grande série, à faible prix de revient, étant donnée la nature des moyens qui sont mis en eouvre. A titre d'exemple, les moyens d'orientation peuvent être réalisés en un matériau tel que de la mousse de polyuréthane, ou par exemple par une ou deux couches de ouate de cellulose renforcée par une feuille de matière plastique présentant des nervures transversales de renforcement.

D'autre part, les moyens de maintien peuvent être réalisés dans des matières traditionnellement utilisées pour des langes, tels que le polychlorure de vinyl.

Il faut remarquer que les matériaux mis en oeuvre selon la présente invention doivent présenter une nature transparente aux rayons X, de manière à permettre de radiographier l'enfant en position.

Le lange selon l'invention, tel que cela a été décrit précédemment, est associé à des moyens d'absorption. De préference, le lange est jeté après chaque usage, ou après un nombre déterminé mais réduit d'usages, au-delà duquel les moyens d'orientation ne présentent plus une rigidité suffisante.

Ainsi, le lange selon la présente invention pourra se substituer aux langes traditionnels, et donc présenter un aspect préventif vis-à-vis d'éventuelles anomalies d'une ou des hanches d'un enfant.

La mise en place du lange selon l'invention est particulièrement simple, et pourra être effectuée par une maman, sans connaissance médicale particulière.

D'autre part, l'aspect extérieur du lange selon l'invention est sensiblement semblable aux langes traditionnels, et il peut être recouverte par des vêtements de l'enfant. Ainsi, dans le case où le lange est utilisé à titre curatif, il présente, sur l'entourage de l'enfant, un effet psychologique nettement moins défavorable que celui provoqué par les dispositifs spécalisés actuellement existants.

La détermination des dimensions pour les différents moyens mis en oeuvre est à la portée de l'Homme de l'Art. Ces dimensions dépendent principalement de la morphologie de l'enfant, et de sa crossance. Les langes selon l'invention, comme les langes traditionnels, peuvent être commercialisés sous différentes tailles, correspondants respectivement à des limits inférieure et supérieure de l'âge de l'enfant.

**Revendications**

1. Lange pour enfant en bas âge destiné à prévenir ou guérir une anomalie d'une hanche ou de deux hanches, telle qu'une dysplasie, une sublaxation de la hanche ou une luxation réductible et stable sous langeage en abduction, ce lange étant constitué par des moyens d'orientation (3, 15) et par des moyens de maintien souples se presentant sous la forme d'une pièce de tissue ou de plastique souple comprenant deux extrémités latérales effilées (13, 14) qui peuvent être nouées autour de la taille de l'enfant et d'une ceinture (8, 23) sensiblement inextensible les moyens de maintien (2, 20) assurant le maintien en position sur les cuisses et le bassin de l'enfant des moyens d'orientation (3, 15) lesquel servents à positionner les jambes de l'enfant de façon fléchie à 90 degrés par rapport au bassin et an abduction à un minimum de 60 degrés, caractérisé par le fait que:

— les extrêmités laterales effilées (13, 14) sout prévues pour être mouées dans le dos de l'enfant,
— les moyens d'orientation sont du type ischio-sacré et constitués essentiellement d'un élément transversal unique en forme de traverse (3) ou de coussin (15) en matériau rigide ou semi-rigide,
— ledit élément transversal (3, 15) est cintré selon une courbe qui correspond sensiblement à l'angle d'ouverture des cuisses de l'enfant que l'on désire obtenir,
— la longueur de l'élément transversal (3, 15) est prévue sensiblement égale à la distance séparant les genoux de l'enfant lorsque ses cuisses sont en abduction de telle sorte que ledit élément (3, 15) serve d'appui aux faces postérieures des cuisses de l'enfant,
— les moyens de maintien (2, 20) sont conformés de manière, en cours d'utilisation, à appuyer l'élément transversal (3, 15) sur les faces postéro-inférieures des cuisses de l'enfant et orienter ainsi les cuisses sensiblement parallèlement aux extrémités dudit élément.

2. Lange selon la revendication 1, caractérisé par le fait que les moyens de maintien (2, 20) comprennent dans leur partie postérieure ladite ceinture (8, 23) sensiblement inextensible, qu'est fermée par des moyens de fermeture autour de la taille de l'enfant, et que l'élément traversal (3, 15) est relié à la ceinture (8, 23) par une languette (10, 24) definissant leur positionnement relatif.

3. Lange selon la revendication 1, caractérisé par le fait que les moyens d'orientation du type ischio-sacré sont situés dans une poche des moyens de maintien (20) collés ou soudés à ceux-ci.

**Patentansprüche**

1. Windel für Säuglinge, zur Verhütung oder Heilung einer Anomalie bei einer oder bei beiden Hüften, wie zum Beispiel eine Dysplasie, einer Sublaxation, oder einer einrenkbaren und bei Wicklung in einer abduzierten Position stabilen Luxation aus Ausrichtmitteln (3, 15), und aus nachgiebigen Haltemitteln in Form eines nachgiebigen Stoff- oder Kunststoffteils mit zwei schmaler werdenden seitlichen Enden (13, 14), die um die Taille des Kindes geknotet werden können, sowie aus einem im wesentlichen undehnbaren Gürtel (8, 23), wobei die Haltemittel (2, 20) sicherstellen, daß die Ausrichmittel (3, 15) in ihrer Postion auf den Schenkeln und dem Becken des Kindes gehalten werden, und die Ausrichtmittel (3, 15) dazu dienen, die Beine des Kindes in einer gegenüber dem Becken unter 90 Grad angewinkelten und um mindestens 60 Grad abduzierten Position zu halten, dadurch gekennzeichnet, daß:

— die schmaler werdenden seitlichen Enden (13, 14) dazu bestimmt sind, auf dem Rücken des Kindes zusammengeknotet zu werden;
— die Ausrichtmittel vom ischiosakralen Typ sind und im wesentlichen aus einem einzelnen transversalen Element in Form einer Traverse (3) oder eines Kissens (15) aus starrem oder halbstarrem Material bestehen;
— das besagte transversale Element (3, 15) gemäß einer Kurve gewölbt ist, die im wesentlichen des gewünschten Öffnungswinkel der Schenkel des Kindes entspricht;
— die Länge des transversalen Elementes (3, 15) im wesentlichen gleich dem Abstand der Knie des Kindes ist, wenn seine Schenkel in der abduzierten Position sind, so daß das besagte Element (3, 15) als Auflage für die hinteren Flächen der Schenkel des Kindes dient;
— die Haltemittel (2, 20) so geformt sind, daß das transversale Element (3, 15) gegen die hinteren unteren Flächen der Schenkel des Kindes gedrückt wird, und so die Schenkel im wesentlichen parallel zu den Enden des besagten Elementes ausgerichtet werden.

2. Windel gemäß Anspruch 1, dadurch gekennzeichnet, daß die Haltemittel (2, 20) in ihrem hinteren Teil den im wesentlichen undehnbaren Gürtel (8, 23) aufweisen der durch Verschlußmittel um die Taille des Kindes gelegt wird, und daß das transversale Element (3, 15) über eine Zunge (10, 24) mit dem Gürtel (8, 23) verbunden ist, wobei diese Zunge (10, 24) deren relative Positionierung festlegt.

3. Windel gemäß Anspruch 1, dadurch gekennzeichnet, daß die Ausrichtmittel vom ischiosakralen Typ in einer Tasche der Haltemittel (20) angeordnet sind, oder auf diese aufgeklebt oder aufgeschweißt sind.

**Claims**

1. Napkin for infant intended for preventing and healing an anomaly of a hip or two hips, such as dysplasy, a subluxation of a hip or a luxation stable and reducible under binding up the infant in its napkin in abduction, this napkin being composed of orientation means (3, 15) and flexible holding means presenting themselves in the form of a flexible piece of tissue or plastic comprising two tapered lateral ends (13, 14) which can be tied around the child waist, and of an appreciably inextensible belt (8, 23), the holding means (2, 20) insuring the holding in position on the child thighs and pelvis of the orientation means (3, 15) which serve to position the child legs in a manner bended to 90 degrees with regard to the pelvis and in abduction at a minimum of 60 degrees, characterized in that:

— the tapered lateral ends (13, 14) are provided for being tied in the back of the child,
— the orientation means are of ischio-sacral type and essentially composed of a single transversel element in form of traverse (3) or cushion (15) in rigid or semi-rigid material,
— said transversal element (3, 15) is curved according to a curve which percepitibly corresponds to the opening angle of the child thighs which it is desired to obtain,
— the length of the transversal element (3, 15) is provided appreciably equal to the distance separating the child knees when its thighs are in abduction in such a way that said element (3, 15) serves as support to the back faces of the child thighs,
— the holding means (2, 20) are conformed in order, during the utilisation, to lean the transversal element (3, 15) against the lower back faces of the child thighs and so to orientate the thighs appreciably in a parallel direction with the ends of said element.

2. Napkin according to claim 1, characterized in that the holding means (2, 20) comprise, in their back part, said appreciably inextensible belt (8, 23) which is closed by closing means around the child waist, and the transversal element (3, 15) is connected to the belt (8, 23) by a small tongue (10, 24) defining their relative positioning.

3. Napkin according to claim 1, characterized in that the orientation means of ischio-sacral type are located in a pocket of the holding means (20) or glued or welded to the latter.

**0 050 062**

Fig 1

Fig 2

0 050 062

Fig 3

Fig 4

2